# EUROPEAN PATENT APPLICATION

(11) **EP 1 130 390 A1**
(43) Date of publication of application: **05.09.2001**
(21) Application number: 00957017.7
(22) Date of filing: 07.09.2000
(51) Int. Cl.: G01N 27/447, C12Q 1/00, C12N 9/00

(54) **PROCESS FOR PRODUCING LIPID-MODIFIED ENZYME AND BIOSENSOR**

(30) Priority: 13.09.1999 JP 25957999
(71) Applicant: MATSUSHITA ELECTRIC INDUSTRIAL CO., LTD., Kadoma-shi, Osaka 571-8501 (JP)
(72) Inventor: TSUJI, Satoko, Katano-shi, Osaka 576-0036 (JP); YOSHIOKA, Toshihiko, Hirakata-shi, Osaka 573-0035 (JP); NANKAI, Shiro, Hirakata-shi, Osaka 573-0071 (JP); MIYAMOTO, Yoshiko, Suita-shi, Osaka 565-0821 (JP)
(74) Representative: Nachshen, Neil Jacob
(86) International application number: JP0006111
(87) International publication number: WO0120316

(57) **Abstract**

The present invention provides a biosensor capable of accurately measuring the concentration of a substrate, such as glucose, with high storage reliability.

A method for producing a lipid-modified enzyme of the present invention includes the steps of: adding an enzyme to a medium containing a lipid represented by the following formula (1) so as to produce a complex of the lipid and enzyme; precipitating the produced complex; and drying the precipitate. A biosensor according to the present invention comprises an electrode system including a working electrode and a counter electrode formed on an electrically insulating base plate; and a reaction layer formed on or in the vicinity of the electrode system, and the reaction layer includes at least two layers: a layer containing a lipid-modified enzyme obtained by the above method and a layer containing an electron mediator. 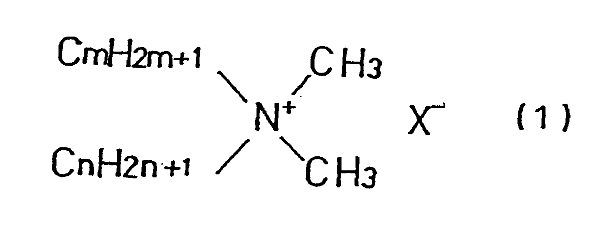 wherein each of m and n is independently an integer between 14 and 18, and X⁻ is Cl⁻, Br⁻, I⁻, H₂PO₄⁻, HSO₄⁻, or NO₃⁻.

## Description

### Technical Field

The present invention relates to a biosensor capable of rapidly and readily quantifying a substrate (specific component) contained in samples such as biological samples, like blood and urine, raw materials and products in the food industry and fruit juice, with high accuracy, and also relates to a method for producing a lipid-modified enzyme for use in the biosensor.

### Background Art

There have been proposed biosensors that readily quantify a specific component (substrate) in biological samples and foodstuffs without diluting and stirring the sample solutions. Japanese Laid-Open Patent Publication No. Hei 3-202764 discloses one example of biosensor in which an electrode system is formed on an insulating base plate by a method such as screen printing and a reaction layer containing an oxidoreductase and an electron mediator (electron acceptor) is formed on this electrode system.

This biosensor quantifies the concentration of the substrate in the sample as follows.

First, the sample solution is dropped on the reaction layer of the biosensor to dissolve the reaction layer and an enzyme reaction proceeds between the substrate in the sample solution and the oxidoreductase in the reaction layer. The electron mediator is reduced in association with this enzyme reaction. After elapse of a certain period of time, a voltage is applied to the electrodes of the sensor to electrochemically oxidize the reduced electron mediator, and the concentration of the substrate in the sample solution can be quantified from an oxidation current value obtained in the oxidation.

A biosensor using a lipid-modified enzyme is disclosed in Japanese Laid-Open Patent Publication No. Hei 7-110313. By modifying an enzyme with a lipid, the enzyme becomes insoluble in water but soluble in an organic solvent, thereby allowing the formation of the reaction layer in a state separated from the water-soluble electron mediator. It is therefore possible to provide a rapid and highly reliable sensor.

In biosensors with conventional structures using no lipid-modified enzyme, since the enzyme and electron mediator are present in a single reaction layer, there is an advantage that a reaction is promptly initiated upon supply of the sample solution. However, when the enzyme is stored in a mixed state with a compound such as an electron mediator for a long period of time, the activity of the enzyme is lowered, resulting in a problem that the response characteristics of the sensors are impaired. There is also a problem that an oxidation current value (blank value) is given for a sample solution containing no substrate. Then, a structure for separating the enzyme and the electron mediator into different layers was considered, but since the enzyme and the electron mediator were both soluble in water, it was difficult to bring them into perfectly separated states.

Meanwhile, as a method for producing a lipid-modified enzyme, a method of mixing a specific lipid dispersion and enzyme solution was proposed (Thin Solid Films, 180. p65 (1989)). However, it was difficult to obtain a highly active lipid-modified enzyme with a high yield.

An object of the present invention is to solve problems as mentioned above while utilizing the advantageous features of biosensors of conventional structures and to provide, a method for producing a highly active lipid-modified enzyme with a high yield.

Moreover, it is an object of the present invention to provide a highly reliable biosensor, which gives a low response value (blank value) when the concentration of a substrate is zero and is capable of quantifying a substrate with high accuracy, by the use of the lipid-modified enzyme.

### Disclosure of Invention

The present invention provides a method for producing a lipid-modified enzyme comprising the steps of: adding an enzyme to a medium containing a lipid dialkyldimethyl ammonium salt represented by the following formula (1) so as to produce a complex of the lipid and enzyme; precipitating the produced complex; and drying the precipitate. 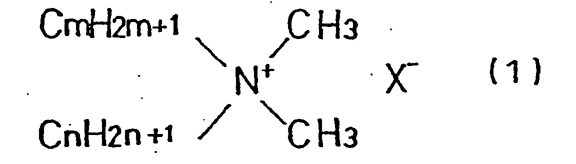 wherein each of m and n is independently an integer between 14 and 18, and X⁻ is Cl⁻, Br⁻, I⁻, H₂PO₄⁻, HSO₄⁻, or NO₃⁻.

When the enzyme is added to the medium containing the lipid, the weight ratio of enzyme to lipid is preferably within a range of 1:0.2 to 1:5.

X⁻ in the above formula (1) is preferably Cl⁻ or Br⁻.

A biosensor of the present invention comprises: an electrically insulating base plate; an electrode system including a working electrode and a counter electrode formed on the base plate; and a reaction layer formed on or in the vicinity of the electrode system, wherein the reaction layer comprises at least a layer containing a lipid-modified enzyme composed mainly of an enzyme and a lipid represented by the above formula (1), and a layer containing an electron mediator.

### Brief Description of Drawings

FIG. 1 is an exploded perspective view of a biosensor according to one example of the present invention, excluding a reaction layer.
FIG. 2 is a schematic vertical sectional view of the biosensor.

### Best Mode for Carrying Out the Invention

The following description will explain a method for producing a lipid-modified enzyme of the present invention.

A lipid represented by the above formula (1) is dispersed or dissolved in a liquid, preferably water, to prepare a lipid dispersion or lipid solution. Next, an enzyme is added to the solution. At this time, it is possible to use either of a method in which the enzyme is added in the form of a solid powder and a method in which a liquid containing the enzyme is added. The solution containing the enzyme and lipid are kept at low temperatures to precipitate a complex of the lipid and enzyme. Next, this precipitate is separated, washed with water, and then dried to obtain a lipid-modified enzyme in the form of powder. Drying is performed at normal temperature or lower temperatures, and a freeze drying method is preferably used. The most preferable medium to be used here is water. It is also possible to use organic solvents including alcohols such as methanol, ethanol and butanols; ethers such as ethyl ether; and acetone. Since these organic solvents do not dissolve the produced lipid-modified enzyme, it is possible to readily separate the produced lipid-modified enzyme from the medium.

Here, in the above formula (1), if both m and n are not greater than 13, the complex of the lipid and enzyme is not produced or the complex is produced with a low yield. On the other hand, if both m and n are not smaller than 19, the lipid dispersion or lipid solution can not be prepared.

Regarding the ratio of the enzyme and the lipid when the enzyme is added to the medium containing the lipid, if the weight ratio of the enzyme to the lipid is within a range of 1:0.2 to 1:5, a yield of no less than 70% can be obtained.

The enzyme modified with the lipid is soluble in specific solvents such as benzene and toluene, but insoluble in water. Therefore, for formation of a layer of enzyme in the biosensor, it is possible to employ a simple method in which the lipid-modified enzyme is dissolved in an organic solvent, dropped on or in the vicinity of the electrodes on the base plate, and dried. Besides, it is possible to form a layer of the water-soluble electron mediator by dropping an aqueous solution thereof on a predetermined position and drying. Hence, if the respective layers are formed according to such a method, irrespective of the order of forming the layer containing the lipid-modified enzyme and the layer containing the electron mediator, the enzyme and electron mediator contained in the respective layers are not affected by each other, and homogeneous layers can be formed.

In the present invention, examples of the enzyme modified with a lipid in the above-mentioned manner include glucose oxidase, glucose dehydrogenase, lactate oxidase, lactate dehydrogenase, fructose oxidase, cholesterol oxidase, cholesterol dehydrogenase, cholesterol esterase, mutarotase, invertase, ascorbate oxidase, and alcohol oxidase.

The reaction layer of the biosensor in accordance with the present invention contains an electron mediator as mentioned above. Examples of the electron mediator include ferricyanides, p-benzoquinone and its derivatives, phenazine methosulfate, methylene blue, ferrocene and its derivatives. One or more than one kind of such electron mediators are used, and it is particularly preferable to use ferricyanides.

The reaction layer of the biosensor of the present invention may contain a hydrophilic polymer as well as the above-mentioned enzymes and electron mediators. The addition of a hydrophilic polymer to the reaction layer can prevent separation of the reaction layer from the surface of the base plate or electrode system. Moreover, the addition of a hydrophilic polymer has the effect of preventing clacking of the reaction layer surface, and is thus effective for improving the reliability of the biosensor. Examples of such a hydrophilic polymer include carboxymethyl cellulose, hydroxyethyl cellulose, hydroxypropyl cellulose, methyl cellulose, ethyl cellulose, ethylhydroxyethyl cellulose, carboxymethylethyl cellulose, polyvinyl pyrrolidone, polyvinyl alcohol, polyamino acid such as polylysine, polystyrene sulfonate, gelatin and its derivatives, acrylic acid or its derivatives, a polymer of maleic anhydride or maleate, starch and its derivatives. Among them, carboxymethyl cellulose is particularly preferred.

As the method for measuring an oxidation current, there are two types of methods: a two-electrode system using only a measuring electrode and a counter electrode; and a three-electrode system further comprising a reference electrode, and the three-electrode system enables more accurate measurement.

The following description will explain the present invention in great detail by illustrating specific examples.

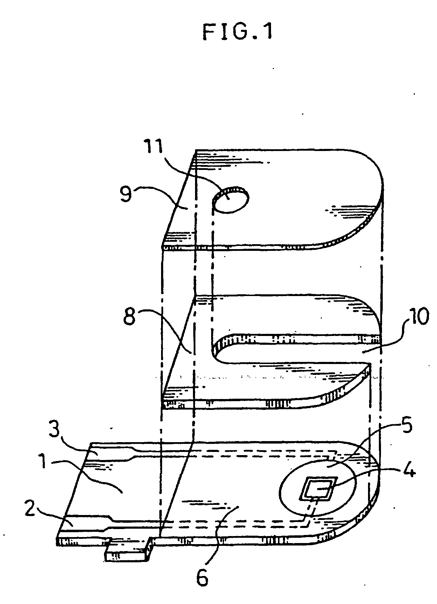

FIG. 1 is an exploded perspective view of a biosensor according to the present invention, excluding the reaction layer. 1 represents an electrically insulating base plate made of polyethylene terephthalate. A silver paste is printed on this base plate 1 by screen printing to form leads 2 and 3. Further, an electrode system including a working electrode 4 and counter electrode 5 made from a conductive carbon paste containing a resin binder, and an electrically insulating layer 6 made from an electrically insulating paste are formed on the base plate 1 by the same printing method. The electrically insulating layer 6 covers the working electrode 4 and counter electrode 5 so that the exposed portion of the working electrode 4 and counter electrode 5 has a certain area, and also covers the leads partially.

As the materials of the leads and electrodes, while platinum, gold, palladium, etc. may be used as well as silver and carbon, leads made from a silver paste and electrodes composed of the base made from a silver paste and covered with a carbon paste are suitable for a disposable type biosensor.

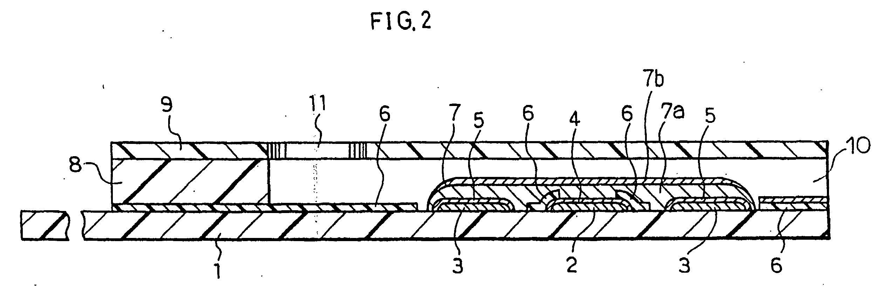

FIG. 2 is a vertical sectional view of the biosensor of the present invention.

A reaction layer 7 is produced by forming a first layer 7a containing an electron mediator on the electrically insulating base plate 1 on which the electrode system is formed as shown in FIG. 1 and forming a second layer 7b containing enzymes on the first layer 7a.

This biosensor is assembled by the insulating base plate 1 made of polyethylene carbonate, a cover 9, and a spacer 8 sandwiched between the base plate 1 and the cover 9. These members are adhered together in a positional relationship as shown by the broken lines of FIG. 1 so as to construct the biosensor.

The spacer 8 has a slit 10 that forms a sample solution supply path, and an air vent 11 is formed in the cover 9. When the base plate 1 and the cover 9 are layered and adhered together with the spacer 8 therebetween, a cavity serving as the sample supply path is formed in the section of the slit 10 between the base plate 1 and cover 9. In this cavity, the open end of the slit 10 as the beginning end serves as a sample solution supply opening, and the final end is connected to the air vent 11.

Here, while a cover member is constructed by a combination of the cover 9 and spacer 8, it may be constructed by a single member provided with a groove equivalent to the slit 10 of the spacer 8. In a biosensor constructed by combining the insulating base plate with the cover member that forms the sample solution supply path between the base plate and the cover member as described above, it is not necessarily to form the reaction layer on the electrode system. The reaction layer needs to be formed at a position where it is exposed to the sample solution supply path and dissolved in the sample solution before the sample solution reaches the electrode system through the sample supply path by capillarity.

### Example 1

An aqueous solution of potassium ferricyanide was dropped on the electrode system on the base plate 1 of FIG. 1 and dried to form the first layer 7a. The amount of potassium ferricyanide contained in this first layer 7a was 1 mg per square centimeter.

Next, a layer of an enzyme modified with a lipid was formed on the first layer 7a as follows.

First, the lipid-modified enzyme was produced by the following method. 10 mg of lipid dioctadecyldimethyl ammonium chloride represented by the formula (1), wherein m=18 and n=18, was mixed with 10 ml of deionized water, and the lipid was dispersed in the deionized water by using an ultrasonic washer. 10 mg of glucose oxidase (EC1.1.3.4: hereinafter referred to as "GOD") was dissolved in this dispersion. After cooling the resultant solution in an ice bath for two hours, it was cooled in a refrigerator for 24 hours. As a result of this cooling, a complex of the lipid and enzyme was precipitated and deposited. After separating this precipitate and washing it with deionized water, it was dried using a freeze-drier. In this manner, the lipid-modified enzyme in the form of powder was obtained. The yield was 99%, and the ratio of the enzyme protein in the lipid-modified enzyme was 50% by weight.

Next, the powder of the lipid-modified enzyme was dissolved in toluene, and the resultant solution was dropped on the above-mentioned first layer 7a and dried to form the second layer 7b. At this time, the layer of the lipid-modified enzyme was formed in such a state that it was completely separated from the previously formed layer of potassium ferricyanide. The amount of lipid-modified enzyme contained in this second layer 7b was 0.2 mg per square centimeter of the reaction layer.

When a sample solution containing glucose is supplied to the reaction layer 7, the glucose in the sample solution is oxidized by the GOD. Then, simultaneously with the oxidation, the electron mediator in the reaction layer is reduced. Subsequently, after a passage of one minute from the supply of the sample solution, a voltage of +0.5 V was applied to the working electrode 4 based on the counter electrode 5 to oxidize the reductant of the electron mediator. Further, five seconds later, the current value was measured. Since the current value is proportional to the concentration of the generated electron mediator, i.e., the concentration of the substrate in the sample solution, it is possible to obtain the glucose concentration in the sample solution by measuring this current value.

Samples with glucose concentrations, of 0, 180, 360 and 540 mg/dl were prepared, and the response current value of the sensor was measured for the respective samples, and the results of the measurements showed that response current value and the glucose concentration had a certain correlation as shown in Table 1 and exhibited good linearity. Further, the blank value was 0 µA.

### Example 2

In the same manner as in Example 1, the first layer 7a containing potassium ferricyanide was formed.

Next, a lipid-modified enzyme was produced by the same method as in Example 1. In this case, 10 mg of lipid dioctadecyldimethyl ammonium chloride represented by the formula (1), wherein m=18 and n=18, 10 ml of deionized water and 5 mg of GOD were used. At this time, the yield of the lipid-modified enzyme was 90%, and the ratio of the enzyme protein in the lipid-modified enzyme was 37% by weight.

The second layer 7b containing the lipid-modified enzyme thus obtained was formed. The amount of lipid-modified enzyme contained in this second layer 7b was 0.3 mg per square centimeter of the reaction layer.

The response current value of the sensor with respect to a glucose standard solution was measured by the same method as in Example 1, and the response current value exhibited good linearity with respect to the glucose concentration as the result of the measurement. Further, the blank value was 0 µA.

### Example 3

In the same manner as in Example 1, the first layer 7a containing potassium ferricyanide was formed.

Next, a lipid-modified enzyme was produced by the same method as in Example 1. In this case, 10 mg of lipid dioctadecyldimethyl ammonium chloride represented by the formula (1), wherein m=18 and n=18, 10 ml of deionized water and 2 mg of GOD were used. At this time, the yield of the lipid-modified enzyme was 82%, and the ratio of the enzyme protein in the lipid-modified enzyme was 20% by weight.

The second layer 7b containing the lipid-modified enzyme thus obtained was formed. The amount of lipid-modified enzyme contained in this second layer 7b was 0.5 mg per square centimeter of the reaction layer.

The response current value of the sensor with respect to the glucose standard solution was measured by the same method as in Example 1, and the response current value exhibited good linearity with respect to the glucose concentration as the result of the measurement. Further, the blank value was 0 µA.

### Example 4

In the same manner as in Example 1, the first layer 7a containing potassium ferricyanide was formed.

Next, a lipid-modified enzyme was produced by the same method as in Example 1. In this case, 5 mg of lipid dioctadecyldimethyl ammonium chloride represented by the formula, (1), wherein m=18 and n=18, 10 ml of deionized water and 10 mg of GOD were used. At this time, the yield of the lipid-modified enzyme was 85%, and the ratio of the enzyme protein in the lipid-modified enzyme was 60% by weight.

The second layer 7b containing the lipid-modified enzyme thus obtained was formed. The amount of lipid-modified enzyme contained in this second layer 7b was 0.2 mg per square centimeter of the reaction layer.

The response current value of the sensor with respect to the glucose standard solution was measured by the same method as in Example 1, and the response current value exhibited good linearity with respect to the glucose concentration as the result of the measurement. Further, the blank value was 0 µA.

### Example 5

In the same manner as in Example 1, the first layer 7a containing potassium ferricyanide was formed.

Next, a lipid-modified enzyme was produced by the same method as in Example 1. In this case, 2 mg of lipid dioctadecyldimethyl ammonium chloride represented by the formula (1), wherein m=18 and n=18, 10 ml of deionized water and 10 mg of GOD were used. At this time, the yield of the lipid-modified enzyme was 70%, and the ratio of the enzyme protein in the lipid-modified enzyme was 76% by weight.

The second layer 7b containing the lipid-modified enzyme thus obtained was formed. The amount of lipid-modified enzyme contained in this second layer 7b was 0.15 mg per square centimeter of the reaction layer.

The response current value of the sensor with respect to the glucose standard solution was measured by the same method as in Example 1, and the response current value exhibited good linearity with respect to the glucose concentration as the result of the measurement. Further, the blank value was 0 µA.

### Comparative Example 1

In order to confirm the effect of lipid modification of the lipid-modified enzyme described in Example 1, the response characteristic and blank value of a sensor using an enzyme that was not modified with a lipid were evaluated. A mixed aqueous solution of potassium ferricyanide and GOD was dropped on the electrode system on the base plate 1 of FIG. 1 and dried to form the reaction layer 7. The amount of potassium ferricyanide contained in this layer 7 was 1 mg per square centimeter of the reaction layer, and the amount of the GOD was 0.1 mg. Since the mixed layer of potassium ferricyanide and GOD was formed for the first layer, the second layer was not formed.

Next, the response current value of the sensor was measured by the same method as in Example 1. As a result, the response current value and the glucose concentration had a certain correlation and exhibited good linearity. This response linearity was almost the same as that shown in Example 1. However, the blank value was 0.1 µA.

Table 1 shows a comparison between the response characteristics of the sensor of Example 1 using the lipid-modified enzyme and the sensor of Comparative Example 1 using an unmodified enzyme.

Next, after storing the sensor of Example 1 and the sensor of Comparative Example 1 in a dried state at 50°C for one month, the blank value was measured in the same manner as above. As a result, with the sensor of Comparative Example 1 using the unmodified enzyme, the blank value was increased to 0.2 µA. On the other hand, with the sensor of Example 1 using the lipid-modified enzyme, the blank value was 0.05 µA.

**Table 1**

| Response current value (µA) | | | | |
|---|---|---|---|---|
| | Glucose concentration (mg/dl) | | | |
| | 0 | 180 | 360 | 540 |
| Example 1 | 0.0 | 6.1 | 12.0 | 18.7 |
| Comparative Example 1 | 0.1 | 5.7 | 12.0 | 18.3 |

### Comparative Example 2

According to the same method as in Example 1, a lipid-modified enzyme was produced using 10 mg of lipid didodecyldimethyl ammonium bromide represented by the formula (1), wherein m=12 and n=12, 10 ml of deionized water and 10 mg of GOD. The yield of the lipid-modified enzyme was not greater than 20%, and the ratio of the enzyme protein in the lipid-modified enzyme was 41% by weight.

Lipid-modified enzymes were produced in the same manner as in Example 1 using lipids having different values of m and n in the formula (1) and 10 mg of enzyme, and the yields and the ratio of the enzyme protein in the respective lipid-modified enzymes were compared as shown in Table 2.

**Table 2**

| m | n | Yield (wt.%) | Ratio of enzyme protein (wt.%) |
|---|---|---|---|
| 12 | 12 | 25 | 44 |
| 14 | 14 | 82 | 57 |
| 16 | 16 | 88 | 70 |
| 18 | 18 | 99 | 50 |

While glucose sensors using glucose oxidase as the enzyme are explained in the above examples, the present invention is also applicable in the same manner to enzymes for use in various sensors, such as lactic acid sensor, cholesterol sensor, fructose sensor, sucrose sensor, alcohol sensor, and ascorbic acid sensor.

### Industrial Applicability

As described above, according to the present invention, it is possible to obtain a biosensor capable of rapidly and readily quantifying a substrate contained in samples such as biological samples, like blood and urine, and raw materials and products used in the food industry, with high accuracy.

## Claims

1. A method for producing a lipid-modified enzyme comprising the steps of:
adding an enzyme to a medium containing a lipid represented by the following formula (1), 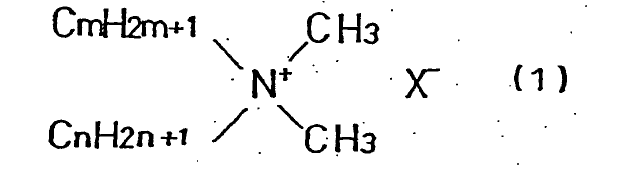
wherein each of m and n is independently an integer between 14 and 18, and X⁻ is Cl⁻, Br⁻, I⁻, H₂PO₄⁻, HSO₄⁻, or NO₃⁻, so as to produce a complex of the lipid and enzyme;
precipitating said produced complex; and
drying said precipitate.

2. The method for producing a lipid-modified enzyme as set forth in claim 1,
wherein a weight ratio of said enzyme and lipid in said medium is within a range of 1:0.2 to 1:5.

3. The method for producing a lipid-modified enzyme as set forth in claim 1,
wherein X⁻ in the formula (1) is Cl⁻ or Br⁻.

4. A biosensor comprising:
an electrically insulating base plate;
an electrode system including a working electrode and a counter electrode formed on said base plate; and
a reaction layer formed on or in the vicinity of said electrode system,
wherein said reaction layer comprises at least a layer containing a lipid-modified enzyme composed mainly of an enzyme and a lipid represented by the following formula (1), 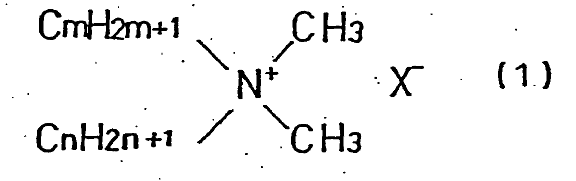 and a layer containing an electron mediator.

5. The biosensor as set forth in claim 4,
wherein X⁻ in the formula (1) is Cl⁻ or Br⁻.
